# FASCICULE DE BREVET EUROPEEN

(11) **EP 3 409 316 B1**
(45) Date de publication et mention de la délivrance du brevet: **23.06.2021**
(21) Numéro de dépôt: 18167034.0
(22) Date de dépôt: 18.11.2009
(51) Int. Cl.: A61M 25/00, A61M 29/02, A61M 5/00, A61M 25/10, A61B 17/22, A61M 25/09

(54) **DISPOSITIF D ANGIOPLASTIE**
ANGIOPLASTIE-VORRICHTUNG
ANGIOPLASTY DEVICE

(30) Priorité: 19.11.2008 FR 0806472
(43) Date de publication de la demande: 05.12.2018
(62) Demande divisionnaire de: 09795455.6
(73) Titulaire: NEXSTEP MEDICAL, 21000 Dijon (FR)
(72) Inventeur: SARRADON, Pierre, 83330 Le Castellet (FR)
(74) Mandataire: Alatis

(56) Documents cités:
- WO-A-2007/132447
- US-A- 4 748 984
- US-A- 4 892 519
- US-A- 4 909 258
- US-A- 5 344 402
- US-A1- 2009 036 831
- US-B1- 6 322 577

## Description

### LIEN AVEC UNE DEMANDE ANTERIEURE

La présente demande est une division de la demande 09795455.

### DOMAINE TECHNIQUE DE L'INVENTION

L'invention se rapporte à un dispositif d'angioplastie destiné à être introduit dans un conduit du corps humain, qui est de préférence un vaisseau sanguin, afin de permettre l'élargissement local du conduit. Ce dispositif est particulièrement utile pour traiter les occlusions ou les sténoses des vaisseaux sanguins.

### ETAT DE LA TECHNIQUE ANTERIEURE

Pour traiter une sténose ou une occlusion d'un vaisseau sanguin, on peut utiliser un dispositif d'angioplastie qui se compose d'une tige sur laquelle est fixé un ballonnet gonflable. Un tel dispositif est par exemple décrit dans le document EP1897584. Ce dispositif comporte une tige pourvue d'un ballonnet gonflable dans laquelle sont percés deux conduits. Le premier conduit est agencé pour recevoir un guide souple filaire permettant de guider le cheminement du dispositif dans le vaisseau jusqu'à la zone à traiter, tandis que le deuxième conduit est en communication fluidique avec le ballonnet permettant ainsi l'injection d'un fluide sous pression dans le ballonnet, en l'occurrence du sérum physiologique, le cas échéant mélangé à un produit de contraste iodé.

Pour mettre en place ce dispositif, on peut injecter un produit de contraste dans le vaisseau sanguin afin de localiser la sténose. Un guide filaire est ensuite introduit dans le vaisseau à traiter afin de faciliter l'insertion du dispositif d'angioplastie au niveau de la sténose. Un guide filaire, ainsi que son procédé d'utilisation, sont par exemple décrits dans le document FR2533130.

Le dispositif d'angioplastie est ensuite introduit autour du guide filaire de façon à ce que le guide filaire se trouve dans le premier conduit. Une fois que le ballonnet est introduit au niveau de la sténose, un fluide sous pression est injecté dans le ballonnet à l'aide du deuxième conduit. Le gonflement du ballonnet permet d'élargir le vaisseau sanguin au niveau de la sténose. Il est ensuite nécessaire de visualiser à nouveau le vaisseau sanguin pour vérifier que la sténose a bien été supprimée. Pour cela, le cathéter à ballonnet est retiré et remplacé par un cathéter d'angiographie, à extrémité multiperforée, qui est positionné en regard ou en amont de la lésion traitée. Le guide est ensuite retiré et un produit de contraste iodé est injecté dans la lumière du cathéter d'angiographie. Si une nouvelle angioplastie est requise, le guide peut être à nouveau monté sur le cathéter d'angiographie, le cathéter peut être ensuite retiré et remplacé par le cathéter à ballonnet.

Alternativement, par soucis de simplification, le guide filaire peut être retiré et l'injection du produit de contraste peut se faire par la lumière libérée par le guide filaire, pour autant que le diamètre de cette lumière de guidage soit suffisant, sachant que le produit de contraste est visqueux. Dans le cas où la sténose n'a pas totalement été supprimée, le ballonnet doit être repositionné à l'endroit où le vaisseau sanguin est rétréci. Pour cela, un guide filaire doit être réintroduit dans le premier conduit pour permettre le repositionnement du ballonnet au niveau du rétrécissement du vaisseau sanguin. Cette opération de repositionnement du ballonnet peut être réitérée plusieurs fois tant que la sténose n'a pas complètement été supprimée.

Le traitement d'une sténose, ou d'une occlusion, comporte donc une succession de phases de traitement, pendant lesquelles le ballonnet est gonflé, et de phases de visualisation, pendant lesquelles un produit de contraste est injecté. Entre chaque phase de traitement et de visualisation, le praticien doit, pour pratiquer l'injection, soit retirer le ballonnet et le remplacer par un cathéter d'angiographie, soit retirer le guide filaire pour utiliser le canal libéré. Ces introductions et retraits successifs du guide filaire dans le premier conduit sont longues et fastidieuses pour le praticien.

Un autre inconvénient de ces techniques vient du fait que la répartition du produit de contraste dans le vaisseau sanguin se fait toujours dans le sens de circulation du sang, de sorte que si le vaisseau comporte des ramifications entre le point d'injection et le point de la lésion, une partie du produit de contraste est diffusée inutilement dans les vaisseaux adjacents.

En outre, dans les dispositifs de l'art antérieur, le produit de contraste est injecté dans le vaisseau sanguin à l'aide du premier conduit, si bien qu'il est injecté dans la totalité du vaisseau sanguin. De grandes quantités de produit de contraste sont donc injectées dans le sang du patient, ce qui peut être néfaste pour sa santé.

Dans le document US 4 892 519 est illustré un dispositif d'angioplastie comportant un guide filaire destiné à être inséré dans un vaisseau sanguin, un corps tubulaire flexible s'étendant suivant un axe longitudinal entre une extrémité proximale et une extrémité distale et présentant une partie proximale incluant l'extrémité proximale et une partie distale incluant l'extrémité distale. Le corps tubulaire comporte un ballonnet gonflable apte à être gonflé pour augmenter le diamètre interne du vaisseau sanguin et disposé radialement autour du corps tubulaire dans la partie distale, un premier conduit interne étanche s'étendant parallèlement à l'axe longitudinal et débouchant d'une part à l'extrémité proximale et d'autre part à l'extrémité distale, de façon à permettre l'insertion du guide filaire dans le premier conduit et le coulissement du corps tubulaire le long du guide filaire, et un deuxième conduit interne s'étendant parallèlement à l'axe longitudinal débouchant d'une part à l'extrémité proximale et d'autre part dans le ballonnet de façon à permettre l'injection d'un fluide dans le ballonnet.

### EXPOSE DE L'INVENTION

L'invention vise à remédier aux inconvénients de l'état de la technique en proposant un dispositif d'angioplastie transluminale percutanée permettant de visualiser le vaisseau sanguin sans effectuer de manœuvre supplémentaire. L'invention vise aussi à améliorer la qualité de la visualisation, tout en diminuant les quantités de produit de contraste injectées dans le sang du patient.

Pour ce faire, est proposé selon un premier aspect de l'invention, un dispositif d'angioplastie selon la revendication 1. Les premier et deuxième conduits peuvent le cas échéant être coaxiaux.

Suivant un autre aspect de l'invention, celle-ci a trait à un dispositif d'angioplastie selon la revendication 3. Avantageusement, selon un mode de réalisation, le premier conduit est circulaire et il a un diamètre sensiblement égal à celui du guide filaire. De cette façon, le conduit et le guide filaire sont bien en contact et donc le guidage du dispositif d'angioplastie par le guide filaire est facilité. Le troisième conduit peut être subdivisé en trois ou quatre conduits qui communiquent avec le premier conduit et sont distribués radialement autour du premier conduit

Le ou les orifices d'injection permettent d'injecter un produit de contraste dans le sang sans avoir à retirer le guide filaire. Le dispositif d'angioplastie peut donc être repositionné très facilement et très rapidement. L'orifice d'injection se trouve préférentiellement à moins de 5 cm du ballonnet. Dans le cas où le corps tubulaire comporte plusieurs orifices d'injection, l'orifice d'injection le plus éloigné du ballonnet, se trouve préférentiellement à moins de 5 cm du ballonnet.

Le dispositif d'angioplastie selon l'invention peut donc être utilisé pour le diagnostic des sténoses ou des occlusions, et il peut également être utilisé pour leur traitement. Ce dispositif permet également de vérifier que le gonflage du ballonnet a bien permis de supprimer l'occlusion ou la sténose sans avoir à enlever le guide filaire.

Le dispositif d'angioplastie selon l'invention joue donc tout à la fois le rôle de sonde de visualisation et le rôle d'instrument pour le traitement des sténoses ou des occlusions.

Par ailleurs, le produit de contraste est injecté localement grâce à l'orifice d'injection situé à proximité du ballonnet, ce qui permet de visualiser très précisément la zone d'intérêt, tout en injectant une petite quantité de produit de contraste dans le sang du patient.

La qualité de la visualisation du produit de contraste peut également être améliorée en répartissant des orifices d'injection radialement autour du corps tubulaire, en aval et en amont du ballonnet, ce qui permet d'avoir une répartition satisfaisante du produit de contraste, et ce quel que soit le sens de circulation du sang.

Le deuxième conduit est étanche pour éviter que le fluide qui permet de gonfler le ballonnet ne passe dans le sang du patient.

Le dispositif d'angioplastie selon l'invention permet en outre d'injecter le produit de contraste sous forte pression en retirant le guide de la partie distale de façon à disposer d'un orifice d'injection supplémentaire. Par ailleurs, lors du passage du corps tubulaire autour du guide filaire, les frottements sont minimisés. Le dispositif d'angioplastie selon l'invention est également avantageux en termes de fabrication industrielle du corps tubulaire, puisque deux conduits seulement doivent être réalisés dans le corps tubulaire, au lieu des trois conduits si l'on souhaite effectuer le guidage et l'injection par des conduits distincts.

La section du premier conduit doit être choisie de manière à satisfaire à deux contraintes contradictoires : elle doit en effet d'une part être la plus grande possible pour permettre l'injection à haut débit du produit de contraste dont la viscosité est élevée, et d'autre part d'une dimension minimale pour faciliter conserver un diamètre global du dispositif le plus petit possible.

Suivants différents modes de réalisation :
- chaque conduit peut être étanche, afin de contrôler le circuit des fluides dans le dispositif ;
- le corps tubulaire peut comporter une pluralité d'orifices d'injection, en communication fluidique avec le troisième conduit, les orifices d'injection étant répartis radialement autour du corps tubulaire. Cette répartition des orifices d'injection permet une meilleure répartition du milieu de visualisation dans la zone à traiter ;
- le ou les orifices d'injection incluent au moins un orifice d'injection aval situé en aval du ballonnet;
- le ou les orifices d'injection incluent au moins un orifice d'injection amont situé en amont du ballonnet. La répartition des orifices d'injection en amont et en aval du ballonnet permet d'améliorer la visualisation de la sténose, quel que soit le sens de circulation du sang ;
- l'extrémité proximale du corps tubulaire est pourvue de trois embouts, chaque embout étant relié de manière étanche à un des conduits.

### BREVE DESCRIPTION DES FIGURES

D'autres caractéristiques et avantages de l'invention ressortiront à la lecture de la description qui suit, en référence aux figures annexées, qui illustrent :
- la figure 1, une vue schématique d'un dispositif d'angioplastie n'entrant pas dans le champ de protection des revendications;
- la figure 2, une vue en coupe transversale du corps tubulaire du dispositif d'angioplastie de la figure 1 ;
- la figure 3, une vue en coupe transversale du corps tubulaire d'un dispositif d'angioplastie selon un deuxième mode de réalisation n'entrant pas dans le champ de protection des revendications;
- la figure 4, une vue en coupe transversale du corps tubulaire d'un dispositif d'angioplastie selon un troisième mode de réalisation n'entrant pas dans le champ de protection des revendications ;
- la figure 5, une vue en coupe transversale du corps tubulaire d'un dispositif d'angioplastie selon un quatrième mode de réalisation de l'invention ;
- la figure 6, une vue en coupe transversale du corps tubulaire d'un dispositif d'angioplastie selon un cinquième mode de réalisation de l'invention ;
- la figure 7, une vue en coupe transversale du corps tubulaire d'un dispositif d'angioplastie selon un sixième mode de réalisation de l'invention ;
- la figure 8, une vue schématique d'un dispositif d'angioplastie selon un septième mode de réalisation ne formant pas partie de l'invention, et n'entrant pas dans le champ de protection des revendications ;
- la figure 9, une vue schématique d'un dispositif d'angioplastie selon un huitième mode de réalisation de l'invention ;
- la figure 10, une vue en coupe transversale de la partie proximale du dispositif d'angioplastie de la figure 9 ;
- la figure 11, une vue en coupe transversale de l'extrémité de la partie distale du dispositif d'angioplastie de la figure 9 ;
- la figure 12, une vue en coupe transversale de la partie proximale d'un neuvième mode de réalisation de l'invention ;
- la figure 13, une vue schématique d'un dispositif d'angioplastie selon un dixième mode de réalisation.

Pour plus de clarté, les éléments identiques ou similaires sont repérés par des signes de référence identiques sur l'ensemble des figures.

### DESCRIPTION DETAILLEE D'UN MODE DE REALISATION

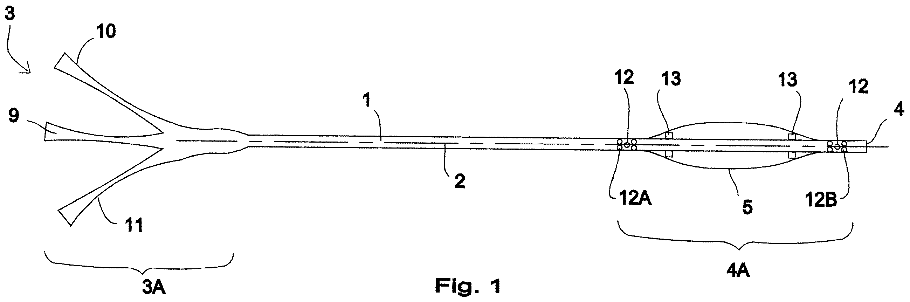

Le dispositif d'angioplastie représenté sur la figure 1 comprend un corps tubulaire 1 réalisé dans un matériau flexible et biocompatible, notamment un matériau synthétique, qui s'étend suivant un axe longitudinal 2 entre une extrémité proximale 3 et une extrémité distale 4. Le corps tubulaire comprend en outre une partie proximale 3A incluant l'extrémité proximale 3 et une partie distale 4A incluant l'extrémité distale 4. Le corps tubulaire 1 est pourvu dans sa partie distale 4A d'un ballonnet 5 gonflable qui entoure radialement le corps tubulaire.

Le corps tubulaire 1 comporte en outre des orifices d'injection 12 percés dans la paroi du corps tubulaire et qui sont disposés radialement en amont (12B) et en aval (12A) du ballonnet gonflable (5). Ces orifices d'injection 12 sont disposés à proximité du ballonnet gonflable. Les termes amont et aval renvoient au sens d'écoulement du fluide dans le dispositif d'angioplastie.

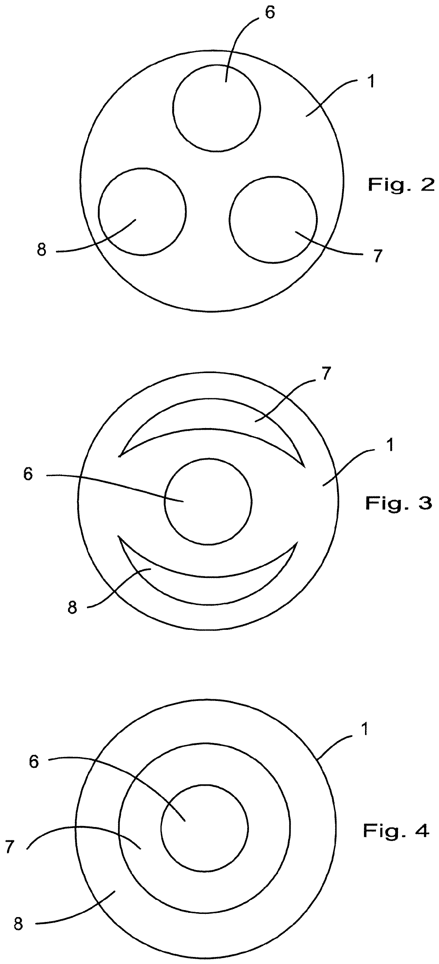

Le corps tubulaire est traversé par trois conduits 6, 7, 8, représentés en coupe sur la figure 2. Les trois conduits 6, 7 et 8 s'étendent parallèlement à l'axe longitudinal. Le premier conduit 6 s'étend sur toute la longueur du corps tubulaire de sorte qu'il relie les extrémités proximale 3 et distale 4 du corps tubulaire. Le premier conduit 6 est de section circulaire et il présente un diamètre intérieur sensiblement égal au diamètre extérieur du guide filaire qui permettra la mise en place du dispositif d'angioplastie dans la veine. Ainsi, le dispositif d'angioplastie est mieux guidé par le guide filaire. L'extrémité proximale 3 est en outre pourvue d'un embout 9 qui est relié au premier conduit 6. L'introduction du guide filaire dans le premier conduit 6 a lieu par l'embout 9.

Le deuxième conduit 7 relie de manière étanche l'extrémité proximale 3 du corps tubulaire au ballonnet gonflable 5. L'extrémité proximale 3 est pourvue d'un deuxième embout 10 qui est relié de manière étanche au deuxième conduit. L'embout 10 est destiné à être relié à un dispositif permettant d'injecter un fluide sous pression dans le deuxième conduit 7 afin de gonfler le ballonnet. Avantageusement, ce fluide sous pression est un mélange de sérum physiologique et de produit de contraste.

Le troisième conduit 8 relie l'extrémité proximale 3 du corps tubulaire aux orifices d'injection 12. L'extrémité proximale 3 est en outre pourvue d'un troisième embout 11 qui est connecté de manière étanche au troisième conduit 8. Le troisième conduit 8 permet l'injection d'un produit de contraste dans le vaisseau sanguin afin de visualiser le rétrécissement du vaisseau sanguin à traiter et le contrôler après l'angioplastie.

En outre, afin de faciliter le positionnement du ballonnet à l'endroit du rétrécissement, en particulier lorsque le ballonnet n'est pas gonflé, une bague de positionnement 13 est placée dans le ballonnet. Cette bague de positionnement 13 est repérable, tout comme le produit de contraste, au moyen de rayons X. Dans un mode de réalisation préféré, le corps tubulaire peut comprendre deux bagues de positionnement, chacune de ces bagues étant placées à la limite du ballonnet afin de le repérer.

L'agencement des conduits à l'intérieur du corps tubulaire peut varier comme on peut le voir sur les figures 3 à 7. Dans les figures 3 et 4, le premier conduit 6 présente une section circulaire de diamètre sensiblement égal à celui du guide filaire qui sera introduit dans ce premier conduit 6. Dans l'agencement de la figure 3, les deuxième et troisième conduits 7 et 8 sont disposés en demi-lune autour du premier conduit, tandis que dans l'agencement de la figure 4, les trois conduits 6, 7 et 8 sont de section transversale circulaire et ils sont concentriques. Le premier conduit 6 est au centre du corps tubulaire, et il est entouré par le deuxième conduit 7, lui-même entouré par le troisième conduit 8.

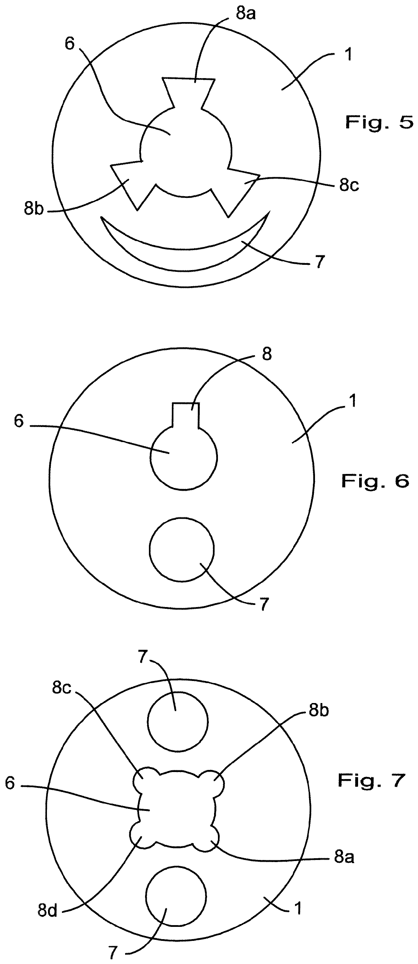

Les figures 5 à 7 présentent un autre agencement dans lequel le premier conduit 6 est ouvert radialement sur le troisième conduit (8). Dans ces modes de réalisation, le premier conduit a toujours une section circulaire de diamètre sensiblement égal à celui du guide filaire afin de permettre un bon guidage du corps tubulaire autour du guide filaire. Afin de permettre un gain de place et de diminuer le nombre de trous à effectuer, le premier conduit 6 est ouvert radialement sur le troisième conduit 8. Sur la figure 5, le troisième conduit est en fait constitué de trois conduits 8a, 8b, 8c qui communiquent avec le premier conduit et qui sont distribués radialement autour du premier conduit. Cette disposition est particulièrement avantageuse lorsque les orifices d'injection 12 sont répartis radialement autour du corps tubulaire puisqu'elle permet une bonne répartition du produit d'injection entre les orifices d'injection 12. De même sur la figure 7, le troisième conduit est en fait constitué de quatre conduits 8a, 8b, 8c, 8d qui communiquent avec le premier conduit et qui sont distribués radialement autour du premier conduit. Ces modes de réalisation permettent en outre de dimensionner le troisième conduit en fonction de la quantité de produit de contraste à injecter, mais aussi en fonction de sa viscosité.

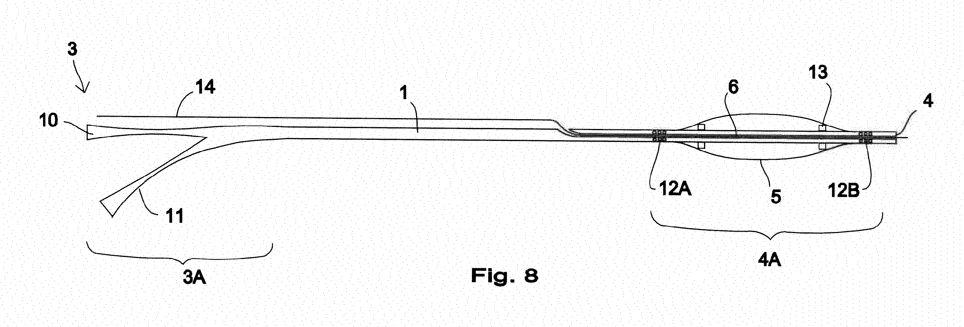

La figure 8 représente un dispositif d'angioplastie selon un autre mode de réalisation ne formant pas partie de l'invention. Dans ce mode de réalisation, le corps tubulaire 1 comprend deux conduits seulement sur dans sa partie proximale 3A, le deuxième et le troisième conduits et il comprend trois conduits dans sa partie distale 4A. Sur cette partie où le corps tubulaire comprend uniquement deux conduits, le guide filaire 14 est extérieur au corps tubulaire 1, tandis que dans la partie distale où le corps tubulaire comprend trois conduits, le guide filaire passe dans le premier conduit.

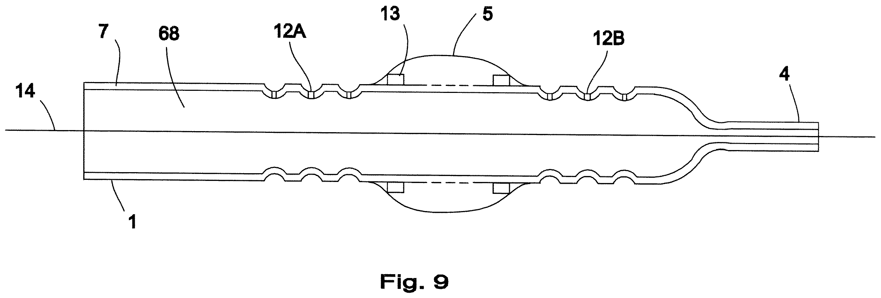

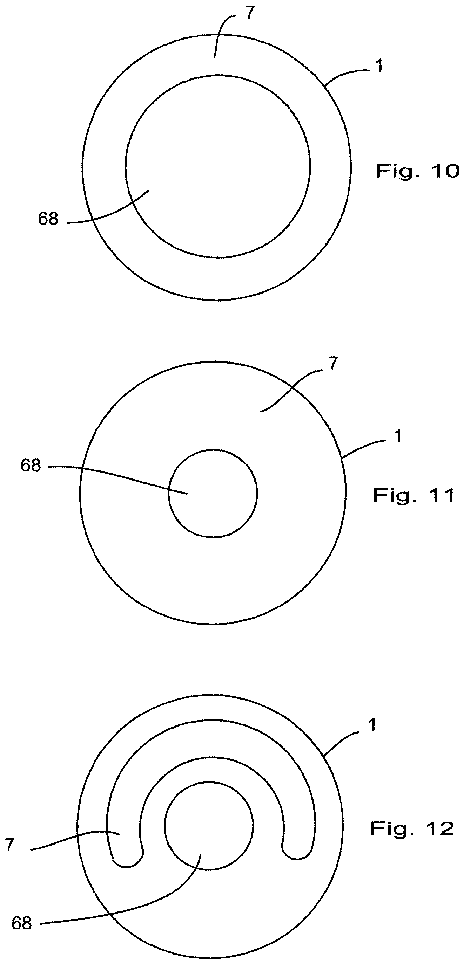

Les figures 9, 10 et 11 représentent un dispositif d'angioplastie selon un autre mode de réalisation, dans lequel le premier conduit 6 et le troisième conduit 8 sont confondus et forment un seul conduit 68. Ce conduit unique 68 a un diamètre supérieur au diamètre extérieur du guide filaire 14 sur toute la longueur du corps tubulaire, à l'exception de l'extrémité de la partie distale 4, dans laquelle le conduit unique a un diamètre sensiblement égal au diamètre extérieur du guide filaire 14. Une coupe transversale de la partie proximale du corps tubulaire de ce dispositif d'angioplastie est représentée sur la figure 10, et une coupe transversale de l'extrémité de la partie distale du corps tubulaire de ce dispositif d'angioplastie est représentée sur la figure 11. Le deuxième conduit 7 et le conduit unique 68 sont concentriques dans ce mode de réalisation. Les orifices d'injection 12 sont de préférence situés en amont de la portion du conduit unique 68 dont le diamètre est égal à celui du corps tubulaire de façon à faciliter le passage du produit de contraste dans le conduit unique 68, sans avoir à retirer le guide filaire. Cet agencement permet d'injecter un produit de contraste sous pression. En outre, il diminue les frottements entre le guide filaire 14 et le conduit unique 68.

La figure 12 représente une variante du mode de réalisation de la figure 9, dans laquelle le conduit unique 68 et le deuxième conduit ne sont pas concentriques.

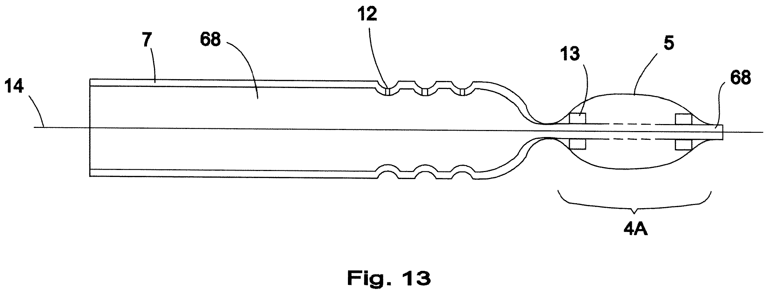

La figure 13 représente une variante du mode de réalisation de la figure 9. Dans ce mode de réalisation, le premier conduit 6 et le troisième conduit 8 sont confondus et forment un seul conduit 68. Ce conduit unique 68 a un diamètre supérieur au diamètre extérieur du guide filaire 14 sur toute la longueur du corps tubulaire, à l'exception de la partie distale 4A, dans laquelle le conduit unique 68 a un diamètre sensiblement égal au diamètre extérieur du guide filaire 14. En outre, dans ce mode de réalisation, le ballonnet 5 est positionné à l'extrémité distale 4A du corps tubulaire, autour de la partie du conduit unique 68 qui a un diamètre sensiblement égal au diamètre du guide filaire 14. De cette façon, le corps tubulaire a sensiblement le même diamètre sur toute sa longueur. En outre, ce diamètre est relativement réduit.

Le traitement d'une sténose à l'aide du dispositif d'angioplastie de la figure 1 va maintenant être précisé.

Dans un premier temps, le praticien insère un cathéter dans la veine à traiter et il injecte un produit de contraste, comme par exemple de l'iode, dans le vaisseau sanguin à traiter. Grâce à ce produit de contraste, le praticien peut observer le vaisseau sanguin en radiographie et il peut ainsi localiser la sténose à traiter. Le praticien introduit ensuite le guide filaire dans le vaisseau à traiter, puis il insert le dispositif d'angioplastie dans le vaisseau sanguin en faisant glisser le premier conduit sur le guide filaire. Le guide filaire facilite l'introduction du dispositif d'angioplastie malgré la souplesse et la longueur de ce dernier.

Le praticien place le ballonnet au niveau du rétrécissement grâce à la bague de marquage 13 qui est radiomarquée. Une fois que le ballonnet a atteint le segment rétréci du vaisseau sanguin, le praticien gonfle le ballonnet à l'aide du deuxième conduit 7. Les dépôts athéromateux qui rétrécissaient le vaisseau sont alors comprimés contre les parois du vaisseau sanguin, ce qui permet d'augmenter le diamètre de sa lumière interne. Une fois cette opération effectuée, le ballonnet est dégonflé, toujours à l'aide du deuxième conduit 7.

Le praticien injecte ensuite un produit de contraste, à l'aide du troisième conduit, dans le sang du patient sans avoir à effectuer les manipulations nécessaires dans l'art antérieur. L'injection de ce produit de contraste permet au praticien de visualiser le vaisseau sanguin après le gonflement du ballonnet de façon à pouvoir vérifier que le segment rétréci a bien été supprimé. Dans le cas où ce segment rétréci n'a pas été totalement supprimé par le premier gonflement, le praticien peut déplacer le ballonnet pour le repositionner au niveau de la portion rétrécie restante. La détection de cette portion rétrécie restante se fait à l'aide du produit de contraste. Une fois le ballonnet repositionné, le praticien peut regonfler le ballonnet. Il peut ensuite à nouveau vérifier que cette opération s'est bien passée. Le praticien peut réitérer ces opérations d'élargissement/vérification autant de fois que nécessaire, et ce, sans avoir à effectuer de manipulation supplémentaire, ce qui lui permet un gain de temps important, et évite certains incidents possibles lors de la manipulation. En outre, le produit de contraste est injecté localement, ce qui permet de très bien visualiser la zone d'intérêt, sans trop injecter de produit de contraste dans le sang du patient.

Naturellement l'invention n'est pas limitée aux exemples de réalisation ci-dessus, et diverses modifications ou variantes peuvent être envisagées à l'intérieur du champ de protection des revendications. Par exemple, d'autres agencements des trois conduits peuvent être envisagés, à l'intérieur du champ de protection des revendications. On peut également envisager d'ajouter un quatrième conduit dans le corps tubulaire, par exemple pour injecter des médicaments ou des agents actifs dans le sang.

Par ailleurs, le corps tubulaire peut soit être un cylindre plein qui est percé par trois conduits, ou alors il peut se présenter sous la forme d'une paroi externe cylindrique qui entoure les trois conduits.

## Revendications

1. Dispositif d'angioplastie comportant :
- un guide filaire destiné à être inséré dans un vaisseau sanguin,
- un corps tubulaire (1) flexible s'étendant suivant un axe longitudinal (2) entre une extrémité proximale (3) et une extrémité distale (4) et présentant une partie proximale (3A) incluant l'extrémité proximale (3) et une partie distale (4A) incluant l'extrémité distale (4), le corps tubulaire (1) comportant:
- un ballonnet gonflable (5) apte à être gonflé pour augmenter le diamètre interne du vaisseau sanguin et disposé radialement autour du corps tubulaire (1) dans la partie distale (4A);
- un premier conduit (68) interne étanche s'étendant parallèlement à l'axe longitudinal (2) et débouchant d'une part à l'extrémité proximale et d'autre part à l'extrémité distale, de façon à permettre l'insertion du guide filaire dans le premier conduit et le coulissement du corps tubulaire le long du guide filaire ;
- un deuxième conduit (7) interne s'étendant parallèlement à l'axe longitudinal (2) débouchant d'une part à l'extrémité proximale (3) et d'autre part dans le ballonnet (5) de façon à permettre l'injection d'un fluide dans le ballonnet;
**caractérisé en ce que** le premier conduit (68) interne a un diamètre supérieur au diamètre extérieur du guide filaire dans la partie proximale du corps tubulaire, et un diamètre sensiblement égal au diamètre extérieur du guide filaire dans l'extrémité distale du corps tubulaire, le corps tubulaire (1) comportant en outre un ou plusieurs orifices d'injection (12) situés dans la partie distale (4A) et débouchant dans le premier conduit (8) interne en amont de la portion premier conduit (68) interne dont le diamètre est sensiblement égal à celui du guide filaire, de façon à permettre l'injection d'un produit de contraste à travers le ou les orifices d'injection (12) lorsque le guide filaire est dans le premier conduit (68) interne.

2. Dispositif d'angioplastie selon la revendication 1, **caractérisé en ce que** les premier et deuxième conduits (68, 7) sont coaxiaux.

3. Dispositif d'angioplastie comportant :
- un guide filaire destiné à être inséré dans un vaisseau sanguin,
- un corps tubulaire (1) flexible s'étendant suivant un axe longitudinal (2) entre une extrémité proximale (3) et une extrémité distale (4) et présentant une partie proximale (3A) incluant l'extrémité proximale (3) et une partie distale (4A) incluant l'extrémité distale (4), le corps tubulaire (1) comportant:
- un ballonnet gonflable (5) apte à être gonflé pour augmenter le diamètre interne du vaisseau sanguin et disposé radialement autour du corps tubulaire (1) dans la partie distale (4A);
- un premier conduit (6) interne étanche s'étendant parallèlement à l'axe longitudinal (2) au moins dans la partie distale (4A) de façon à permettre l'insertion du guide filaire dans le premier conduit et le coulissement du corps tubulaire le long du guide filaire ;
- au moins un deuxième conduit (7) interne s'étendant parallèlement à l'axe longitudinal (2), débouchant d'une part à l'extrémité proximale (3) et d'autre part dans le ballonnet (5) de façon à permettre l'injection d'un fluide dans le ballonnet;
**caractérisé en ce que** le corps tubulaire (1) comporte en outre:
- un ou plusieurs orifices d'injection (12) situés dans la partie distale (4A) ;
- au moins un troisième conduit (8) interne, le premier conduit étant ouvert radialement sur le troisième conduit, le troisième conduit débouchant d'une part à l'extrémité proximale (3) et d'autre part dans le ou les orifices d'injection (12) de façon à permettre l'injection d'un produit de contraste à travers le ou les orifices d'injection (12) lorsque le guide filaire est dans le premier conduit.

4. Dispositif d'angioplastie selon la revendication 3, **caractérisé en ce que** le premier conduit a une section circulaire de diamètre sensiblement égal à celui du guide filaire permettant un guidage du corps tubulaire autour du guide filaire.

5. Dispositif d'angioplastie selon la revendication 3 ou la revendication 4, **caractérisé en ce que** le troisième conduit est subdivisé en trois ou quatre conduits qui communiquent avec le premier conduit et sont distribués radialement autour du premier conduit.

6. Dispositif d'angioplastie selon l'une quelconque des revendications précédentes, **caractérisé en ce que** les orifices d'injection (12) sont répartis radialement autour du corps tubulaire (1).

7. Dispositif d'angioplastie selon l'une quelconque des revendications précédentes, **caractérisé en ce que** le ou les orifices d'injection (12) incluent au moins un orifice d'injection amont (12B) situé en amont du ballonnet (5).

8. Dispositif d'angioplastie selon la revendication 7, **caractérisé en ce que** le ballonnet (5) est positionné à l'extrémité distale (4A) du corps tubulaire, autour de la partie du premier conduit (68) qui a un diamètre sensiblement égal au diamètre du guide filaire (14).

9. Dispositif d'angioplastie selon l'une quelconque des revendications 1 ou 2, le cas échéant en combinaison avec l'une quelconque des revendications 6 à 7, **caractérisé en ce que** le ou les orifices d'injection (12) incluent au moins un orifice d'injection aval (12B) situé en aval du ballonnet (5).

10. Dispositif d'angioplastie selon l'une quelconque des revendications précédentes, **caractérisé en ce que** les premier et deuxième conduits (6, 68, 7) sont disposés côte à côte.

11. Dispositif d'angioplastie selon l'une quelconque des revendications précédentes, **caractérisé en ce que** le premier conduit (6, 68) est de section circulaire.

12. Dispositif d'angioplastie selon l'une quelconque des revendications précédentes, **caractérisé en ce que** l'orifice d'injection le plus éloigné du ballonnet parmi le ou les orifices d'injection se trouve à moins de 5 cm du ballonnet.

## Patentansprüche

1. Angioplastievorrichtung, umfassend:
- eine Drahtführung, zum Einführen in einem Blutgefäß,
- einen flexiblen röhrenförmigen Körper (1), der sich entlang einer Längsachse (2) zwischen einem proximalen Ende (3) und einem distalen Ende (4) erstreckt und einen proximalen Teil (3A) mit dem proximalen Ende (3) und einen distalen Teil (4A) mit dem distalen Ende (4) aufweist, wobei der röhrenförmige Körper (1) umfasst:
- einen aufblasbaren Ballon (5), der aufblasbar ist, um den Innendurchmesser des Blutgefäßes zu vergrößern, und radial um den röhrenförmigen Körper (1) im distalen Teil (4A) angeordnet ist;
- einen ersten abgedichteten inneren Kanal (68), der sich parallel zur Längsachse (2) erstreckt und einerseits am proximalen Ende und andererseits am distalen Ende derart mündet, dass das Einführen der Drahtführung in den ersten Kanal und das Gleiten des röhrenförmigen Körpers entlang der Drahtführung möglich sind;
- einen zweiten inneren Kanal (7), der sich parallel zur Längsachse (2) erstreckt und einerseits am proximalen Ende (3) und andererseits in den Ballon (5) derart mündet, dass die Injektion einer Flüssigkeit in den Ballon möglich ist;
**dadurch gekennzeichnet, dass** der erste innere Kanal (68) einen Durchmesser aufweist, der größer als der Außendurchmesser der Drahtführung im proximalen Abschnitt des röhrenförmigen Körpers ist, und einen Durchmesser, der im Wesentlichen gleich dem Außendurchmesser der Drahtführung am distalen Ende des röhrenförmigen Körpers ist, wobei der röhrenförmige Körper (1) ferner eine oder mehrere Injektionsöffnungen (12) umfasst, die sich im distalen Teil (4A) befinden und in den ersten inneren Kanal (8) stromaufwärts des Abschnitts des ersten inneren Kanals (68), dessen Durchmesser im Wesentlichen gleich dem der Drahtführung ist, derart münden, dass die Injektion eines Kontrastmittels durch die oder mehrere Einspritzöffnungen (12) möglich ist, wenn sich die Drahtführung im ersten inneren Kanal (68) befindet.

2. Angioplastievorrichtung nach Anspruch 1, **dadurch gekennzeichnet, dass** der erste und der zweite Kanal (68, 7) koaxial sind.

3. Angioplastievorrichtung, umfassend:
- eine Drahtführung, zum Einführen in einem Blutgefäß,
- einen flexiblen röhrenförmigen Körper (1), der sich entlang einer Längsachse (2) zwischen einem proximalen Ende (3) und einem distalen Ende (4) erstreckt und einen proximalen Teil (3A) mit dem proximalen Ende (3) und einen distalen Teil (4A) mit dem distalen Ende (4) aufweist, wobei der röhrenförmige Körper (1) umfasst:
- einen aufblasbaren Ballon (5), der aufblasbar ist, um den Innendurchmesser des Blutgefäßes zu vergrößern, und radial um den röhrenförmigen Körper (1) im distalen Teil (4A) angeordnet ist;
- einen ersten abgedichteten inneren Kanal (6), der sich parallel zur Längsachse (2) zumindest in dem distalen Teil (4A) derart erstreckt, dass das Einführen der Drahtführung in den ersten Kanal und das Gleiten des röhrenförmigen Körpers entlang der Drahtführung möglich sind;
- mindestens einen zweiten inneren Kanal (7), der sich parallel zur Längsachse (2) erstreckt und einerseits am proximalen Ende (3) und andererseits im Ballon (5) derart mündet, dass die Injektion einer Flüssigkeit in den Ballon möglich ist;
**dadurch gekennzeichnet, dass** der röhrenförmige Körper (1) ferner umfasst:
- eine oder mehrere Injektionsöffnungen (12), die sich im distalen Teil (4A) befinden;
- mindestens einen dritten inneren Kanal (8), wobei der erste Kanal am dritten Kanal radial offen ist, der dritte Kanal einerseits am proximalen Ende (3) mündet und andererseits in der oder mehreren Injektionsöffnungen (12) derart mündet, dass die Injektion eines Kontrastmittels durch die oder mehrere Injektionsöffnungen (12) möglich sind, wenn sich die Drahtführung im ersten Kanal befindet.

4. Angioplastievorrichtung nach Anspruch 3, **dadurch gekennzeichnet, dass** der erste Kanal einen kreisförmigen Querschnitt mit einem Durchmesser aufweist, der im Wesentlichen dem der Drahtführung entspricht und eine Führung des röhrenförmigen Körpers um die Drahtführung ermöglicht.

5. Angioplastievorrichtung nach Anspruch 3 oder Anspruch 4, **dadurch gekennzeichnet, dass** der dritte Kanal in drei oder vier Kanäle unterteilt ist, die mit dem ersten Kanal kommunizieren und radial um den ersten Kanal verteilt sind.

6. Angioplastievorrichtung nach einem der vorhergehenden Ansprüche, **dadurch gekennzeichnet, dass** die Injektionsöffnungen (12) radial um den röhrenförmigen Körper (1) verteilt sind.

7. Angioplastievorrichtung nach einem der vorhergehenden Ansprüche, **dadurch gekennzeichnet, dass** die oder mehrere Injektionsöffnungen (12) mindestens eine stromaufwärtige Injektionsöffnung (12B) umfassen, die sich stromaufwärts des Ballons (5) befindet.

8. Angioplastievorrichtung nach Anspruch 7, **dadurch gekennzeichnet, dass** der Ballon (5) am distalen Ende (4A) des röhrenförmigen Körpers um den Teil des ersten Kanals (68) angeordnet ist, der einen Durchmesser aufweist, der im Wesentlichen dem Durchmesser der Drahtführung (14) entspricht.

9. Angioplastievorrichtung nach einem der Ansprüche 1 oder 2, gegebenenfalls in Kombination mit einem der Ansprüche 6 bis 7, **gekennzeichnet dadurch, dass** die oder mehrere Injektionsöffnungen (12) mindestens eine Öffnung stromabwärts der Injektion (12B) umfassen, die sich stromabwärts des Ballons befindet (5).

10. Angioplastievorrichtung nach einem der vorhergehenden Ansprüche, **dadurch gekennzeichnet, dass** der erste und der zweite Kanal (6, 68, 7) nebeneinander angeordnet sind.

11. Angioplastievorrichtung nach einem der vorhergehenden Ansprüche, **dadurch gekennzeichnet, dass** der erste Kanal (6, 68) einen kreisförmigen Querschnitt aufweist.

12. Angioplastievorrichtung nach einem der vorhergehenden Ansprüche, **dadurch gekennzeichnet, dass** sich die vom Ballon am weitesten entfernte Injektionsöffnung unter den Injektionsöffnungen weniger als 5 cm vom Ballon entfernt befindet.

## Claims

1. Angioplasty device comprising:
- a guide wire intended to be inserted into a blood vessel,
- a flexible tubular body (1) extending along a longitudinal axis (2) between a proximal end (3) and a distal end (4) and having a proximal part (3A) including the proximal end (3) and a distal part (4A) including the distal end (4), the tubular body (1) comprising:
- an inflatable balloon (5) which is capable of being inflated to increase the internal diameter of the blood vessel and which is disposed radially around the tubular body (1) in the distal part (4A);
- a first leaktight internal conduit (68) extending parallel to the longitudinal axis (2) and opening out on the one hand at the proximal end and on the other hand at the distal end, so as to allow the insertion of the guide wire into the first conduit and the sliding of the tubular body along the guide wire;
- a second internal conduit (7) extending parallel to the longitudinal axis (2) opening out on the one hand at the proximal end (3) and on the other hand in the balloon (5) so as to allow the injection of a fluid into the balloon;
**characterized in that** the first internal conduit (68) has a diameter greater than the external diameter of the guide wire in the proximal portion of the tubular body, and a diameter substantially equal to the external diameter of the guide wire in the distal end of the tubular body, the tubular body (1) further comprising one or more injection orifices (12) located in the distal part (4A) and opening into the first internal conduit (8) upstream of the first internal conduit portion (68), the diameter of which is substantially equal to that of the guide wire, so as to allow the injection of a contrast product through the injection orifice(s) (12) when the guide wire is in the first internal conduit (68).

2. Angioplasty device according to claim 1, **characterized in that** the first and second conduits (68, 7) are coaxial.

3. Angioplasty device comprising:
- a guide wire intended to be inserted into a blood vessel,
- a flexible tubular body (1) extending along a longitudinal axis (2) between a proximal end (3) and a distal end (4) and having a proximal part (3A) including the proximal end (3) and a distal part (4A) including the distal end (4), the tubular body (1) comprising:
- an inflatable balloon (5) which is capable of being inflated to increase the internal diameter of the blood vessel and which is disposed radially around the tubular body (1) in the distal part (4A);
- a first leaktight internal conduit (6) extending parallel to the longitudinal axis (2) at least in the distal part (4A) so as to allow the insertion of the guide wire in the first conduit and the sliding of the tubular body on it along the guide wire;
- at least one second internal conduit (7) extending parallel to the longitudinal axis (2) opening out on the one hand at the proximal end (3) and on the other hand in the balloon (5) so as to allow the injection of a fluid into the balloon;
**characterized in that** the tubular body (1) further comprises:
- one or more injection orifices (12) located in the distal part (4A);
- at least one third internal conduit (8), the first conduit being open radially on the third conduit, the third conduit opening out on the one hand at the proximal end (3) and on the other hand in the injection orifice(s) (12) so as to allow the injection of a contrast product through the injection orifice(s) (12) when the guide wire is in the first conduit.

4. Angioplasty device according to claim 3, **characterized in that** the first conduit has a circular cross section substantially equal to that of the guide wire allowing guidance of the tubular body around the guide wire.

5. Angioplasty device according to claim 3 or claim 4, **characterized in that** the third conduit is subdivided into three or four conduits which communicate with the first conduit and are distributed radially around the first conduit.

6. Angioplasty device according to any of the preceding claims, **characterized in that** the injection orifices (12) are distributed radially around the tubular body (1).

7. Angioplasty device according to any of the preceding claims, **characterized in that** the injection orifice(s) (12) include at least one upstream injection orifice (12B) located upstream of the balloon (5).

8. Angioplasty device according to claim 7, **characterized in that** the balloon (5) is positioned at the distal end (4A) of the tubular body, around the part of the first conduit (68) which has a diameter substantially equal to the diameter of the guide wire (14).

9. Angioplasty device according to any of claims 1 or 2, optionally in combination with any of claims 6 to 7, **characterized in that** the injection orifice(s) (12) include(s) at least one downstream injection orifice (12B) located downstream of the balloon (5).

10. Angioplasty device according to any of the preceding claims, **characterized in that** the first and second conduits (6, 68, 7) are arranged side by side.

11. Angioplasty device according to any of the preceding claims, **characterized in that** the first conduit (6, 68) has a circular section.

12. Angioplasty device according to any of the preceding claims, **characterized in that** the injection orifice furthest from the balloon among the injection orifice(s) is located less than 5 cm from the balloon.
